# EUROPÄISCHE PATENTSCHRIFT

(11) **EP 1 651 123 B1**
(45) Veröffentlichungstag und Bekanntmachung des Hinweises auf die Patenterteilung: **12.01.2011**
(21) Anmeldenummer: 03817900.8
(22) Anmeldetag: 08.08.2003
(51) Int. Cl.: A61B 17/86, F16B 2/06

(54) **KLEMMVORRICHTUNG**
CLAMPING DEVICE
DISPOSITIF DE SERRAGE

(43) Veröffentlichungstag der Anmeldung: 03.05.2006
(73) Patentinhaber: Synthes GmbH, 4436 Oberdorf (CH)
(72) Erfinder: NIEDERBERGER, Alfred, CH-2540 Grenchen (CH); SCHLÄFLI, Christoph, CH-6821 Rovio (TI) (CH); GICQUEL, Philippe Service de Chirurgie Infantile, F-67098 Strasbourg Cedex (FR)
(74) Vertreter: Lusuardi, Werther
(86) Internationale Anmeldenummer: PCT/CH2003/000538
(87) Internationale Veröffentlichungsnummer: WO 2005/013840

(56) Entgegenhaltungen:
- WO-A-01/91660
- GB-A- 2 031 731
- US-A- 630 428
- US-A- 904 341
- US-A- 4 095 914
- US-A- 4 848 953

## Beschreibung

Die Erfindung bezieht sich auf eine Klemmvorrichtung gemäss dem Oberbegriff des Patentanspruchs 1.

In der Pädiatrie besteht ein Bedürfnis Knochenfrakturen nahe der Wachstumsfuge zu versorgen, ohne dass dabei die Wachstumsfuge verletzt wird, da sonst der Knochen nicht mehr weiter wachsen kann. Statt den in der Osteosynthese üblichen Knochenschrauben verwendet man deshalb für diesen Zweck gerne sogenannte Kirschnerdrähte. Sie haben einen sehr geringen Durchmesser und verursachen deshalb - falls überhaupt eine Verletzung der Wachtumsfuge auftritt - nur einen marginalen Schaden. Der Nachteil dieser Operationstechnik besteht aber darin, dass mit einem Kirschnerdraht allein keine Kompression der Knochenfraktur erzeugt werden kann. Es sind deshalb Implantate konstruiert worden, bei denen auf dem implantierten Kirschnerdraht eine kleine Kugel mittels einer Madenschraube quer zum Kirschnerdraht auf diesem fixiert wird, so dass die Endposition des Kirschnerdrahtes gesichert wird. Die in der Kugel befindliche Madenschraube wird mittels eines - über eine Sollbruchstelle fest mit der Madenschraube verbundenen - Instrumentes angezogen, welches bestimmungsgemäss nach erfolgter Fixation abbricht, so dass die vorgenommene Fixierung und Positionierung der Kugel nicht mehr rückgängig gemacht werden kann.

Aus der WO01/91660 A1 BIOTEK ist eine geschlitzte Knochenschraube bekannt. Die Schlitze sind aber nur an ihrem hinteren Kopf-Ende angebracht und nicht an ihrer Spitze, so dass die Knochenschraube nur am hinteren Kopfende komprimierbar ist.

Aus der GB 2 031 731 KNONNER ist eine nicht für die Implantation in den Körper bestimmte, geschlitzte Hohlschraube bekannt, welche ein eingängiges Gewinde trägt und eine glatte Innenbohrung aufweist.

Hier will die Erfindung Abhilfe schaffen. Der Erfindung liegt die Aufgabe zugrunde, eine Klemmvorrichtung zum reversiblen Festklemmen eines longitudinalen Fixationselementes, insbesondere eines Kirschnerdrahtes, zu schaffen.

Die Erfindung löst die gestellte Aufgabe mit einer Klemmvorrichtung, welche die Merkmale des Anspruchs 1 aufweist.

Die durch die Erfindung erreichten Vorteile bestehen darin, dass die Klemmvorrichtung mit der Knochenplatte zusammen in der Lage ist, einen Kirschnerdraht in ihren Durchgangsbohrungen axial verschiebbar aufzunehmen. Der Kirschnerdraht kann dann in der gewünschten Lage positioniert werden und durch Eindrehen der in ihrem . vorderen Bereich kompressibel ausgestaltete Klemmvorrichtung in die Knochenplatte gesichert werden. Dies kann auch - im Gegensatz zu den Vorrichtungen gemäss dem Stand der Technik - mehrmals wiederholt werden, falls man mit der Position des Kirschnerdrahtes noch nicht zufrieden ist. Der Vorteil liegt also darin, das keine laterale Verklemmung mit einer separaten winzigen Madenschraube erfolgt, sondern ohne zusätzliche Bauelemente eine direkte axiale Verklemmung erfolgt, die zudem reversibel ist.

Ein weiterer Vorteil im Vergleich zum Stand der Technik ist der Umstand, dass das Verklemmen entlang der Achse des Kirschnerdrahtes erfolgen kann. Beim Stand der Technik ist es nötig quer zum Kirschnerdraht die Madenschraube einzudrehen, was die Operationstechnik und die Handhabung kompliziert.

Das Anwendungsgebiet der Klemmvorrichtung ist aber nicht auf die Pädiatrie beschränkt, sonder universell, so z.B. auch auf den proximalen Humerus.

Bei einer besonderen Ausführungsform ist das Gewinde der Schraube doppelgängig ausgebildet . Dies erleichtert das Eindrehen der Schraube, da sie in der kegelförmigen Bohrung und dessen komplementärem Innengewinde schneller greift als dies bei eingängigen Gewinden der Fall wäre.

Bei einer weiteren Ausführungsform ist das vordere Ende der Schraube mit drei oder vier Längsschlitzen versehen, welche vorzugsweise in gleichmässigen Abständen angeordnet sind.

Bei einer besonderen Ausführungsform ist die Länge L der Längsschlitze kleiner als die Bauhöhe H der Schraube und es gilt vorzugsweise L < 0,8 H.

Bei einer weiteren Ausführungsform ist die Innenwand der Durchgangsbohrung der Schraube aufgerauht oder makroskopisch strukturiert. Dadurch erzielt man eine verbesserte Klemmwirkung.

Die Klemmvorrichtung umfasst eine Knochenplatte , welche mehrere Plattenbohrungen umfasst, wobei mindestens eine der Plattenbohrungen eine kegelförmige Bohrung aufweist, welche geeignet ist, die Schraube der Klemmvorrichtung aufzunehmen und mindestens im Bereich des vorderen Endes radial zu komprimieren. Statt einer Knochenschraube kann somit auch ein Kirschnerdraht durch das Plattenloch geführt und darin fixiert werden.

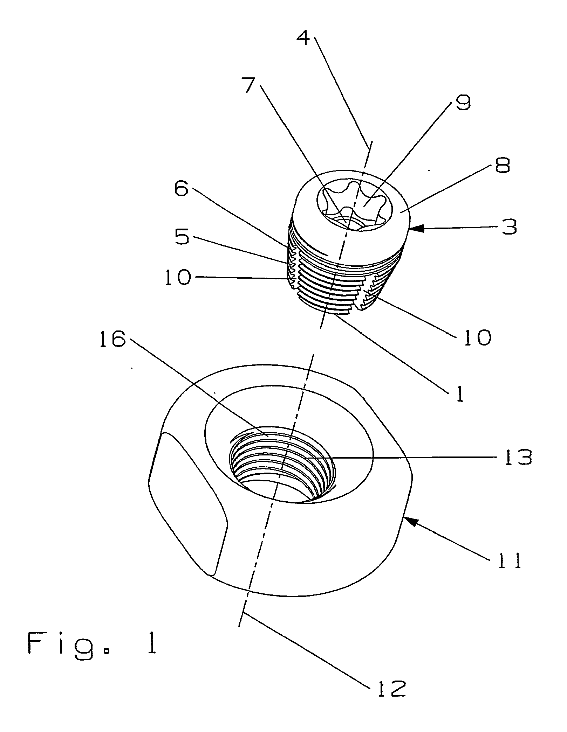

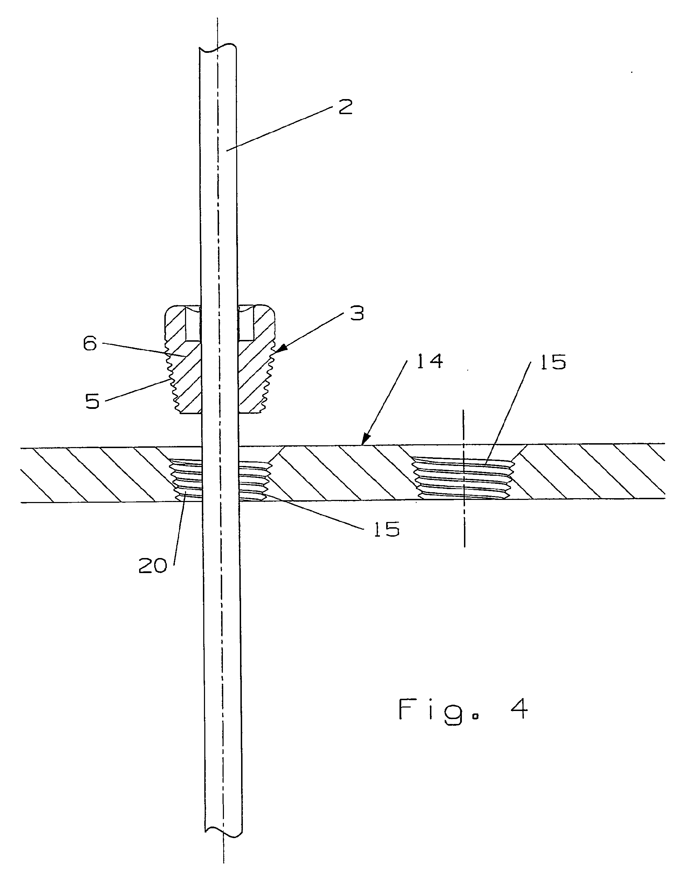

Die Erfindung und Weiterbildungen der Erfindung werden im folgenden anhand der teilweise schematischen Darstellungen mehrere Ausführungsbeispiele noch näher erläutert, wobei nur die in Fig. 4 dargestellte Klemmvorrichtung mit Knochenplatte ein Ausführungsbeispiel der Erfindung ist, während die in den Figs. 1-3 dargestellte Klemmvorrichtung mit Klemmring nicht in den Schutzbereich der Ansprüche fällt.

Es zeigen:
Fig. 1 eine perspektivische Ansicht der Klemmvorrichtung mit einem Klemmring vor dem Verklemmen;
Fig. 2 einen Längsschnitt durch die Klemmvorrichtung mit einem Klemmring nach Fig. 1;
Fig. 3 einen Längsschnitt durch die Klemmvorrichtung mit einem darin eingesetzten Kirschnerdraht nach dem Verklemmen im Klemmring; und.
Fig. 4 einen Längsschnitt durch die Klemmvorrichtung mit einer Knochenplatte und einem Kirschnerdraht.

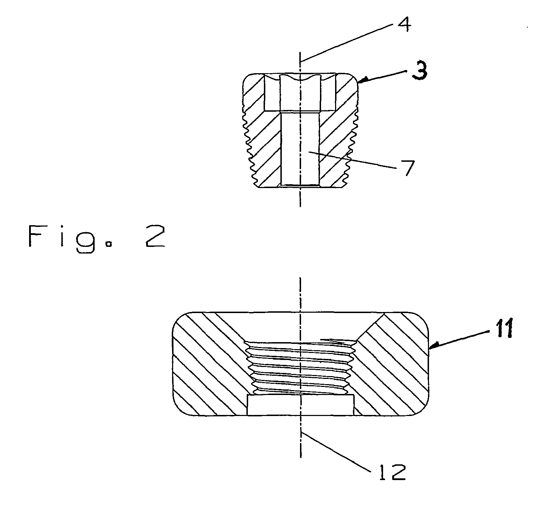

In Fig. 1 und 2 ist eine Ausführungsform der Klemmvorrichtung dargestellt, welche eine teilweise kegelstumpfförmige Schraube 3 mit einer Kegelachse 4 und einen Klemmring 11 mit einer Zentralachse 12 umfasst. Die Schraube 3 ist auf der Mantelfläche 5 mit einem konischen Gewinde 6 versehen, zur Kegelachse 4 koaxial von einer Durchgangsbohrung 7 durchdrungen und mittels vier zur Kegelachse 4 im wesentlichen parallel verlaufenden Längsschlitzen 10 auf einem vorderen Segment der Länge L radial elastisch ausgestaltet. Die Längsschlitze 10 weisen vom vorderen Ende 1 der Schraube 3 gemessen eine Länge L auf, sind am vorderen Ende 1 der Schraube 3 offen und dringen radial von der Mantelfläche 5 her bis in die Durchgangsbohrung 7 ein. Die Längsschlitze 10 sind in einer zur Kegelachse 4 orthogonalen Querschnittsfläche der Schraube 3 betrachtet symmetrisch auf der kreisringförmigen Wand der Schraube 3 verteilt.

Ferner umfasst die Schraube 3 am hinteren Ende 8 ein kreiszylindrisches Segment wo die Mittel 9 zum Eindrehen der Schraube 3 angebracht sind, welche zur Kegelachse 4 koaxial vom hinteren Ende 8 der Schraube 3 her eindringen. Die Mittel 9 zum Eindrehen der Schraube 3 sind hier als Innensechsrund ausgestaltet, können aber jede beliebige geeignete Form aufweisen.

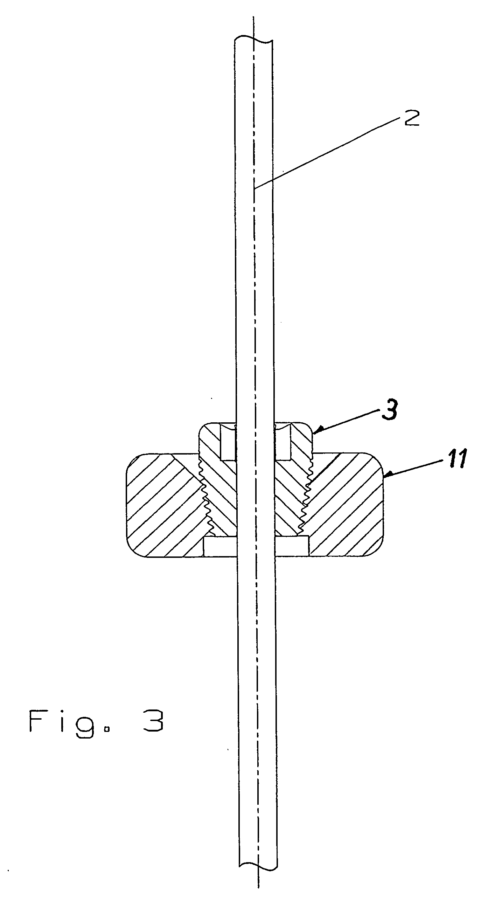

Wie in Fig. 1 dargestellt ist der zusammen mit der Schraube 3 zu verwendende Klemmring 11 aussen koaxial zu seiner Zentralachse 12 kreiszylindrisch ausgestaltet und umfasst eine kegelförmige Bohrung 13, welche ein zum Gewinde 6 der Schraube 3 komplementäres Innengewinde 16 aufweist. Beim Einschrauben der Schraube 3 in den Klemmring 11 wird diese durch die kegelstumpfförmige Ausgestaltung der Schraube 3 und der dazu komplementär ausgestalteten, kegelförmigen Bohrung 13 auf ihrem radial elastischen Segment der Länge L radial komprimiert, wobei auch der zur Kegelachse 4 orthogonale Querschnitt der Durchgangsbohrung 7 verringert wird, so dass ein in der Durchgangsbohrung 7 eingespanntes Fixationselement 2 (Fig. 3), beispielsweise ein Kirschnerdraht in der Schraube 3 fixiert wird.

In Fig. 4 ist eine kegelstumpfförmige Schraube 3 zusammen mit einer Knochenplatte 14 und einem Fixationselement 2, beispielsweise einem Kirschnerdraht dargestellt. Die Schraube 3 unterscheidet sich nicht von der in den Fig. 1 bis 3 dargestellten Ausführungsform. Anstelle des Klemmringes 11 (Fig. 1 - 3) umfasst die Klemmvorrichtung gemäss dieser Ausführung eine Knochenplatte 14, welche mehrere kegelförmige Plattenbohrungen 15 mit konischen Innengewinden 20 enthält. Die konischen Innengewinde 20 sind komplementär zum Gewinde 6 auf der Mantelfläche 5 der Schraube 3 ausgestaltet, so dass je eine Schraube 3 in eine Plattenbohrung 15 einschraubbar ist. Genau wie bei der in den Fig. 1 - 3 dargestellten Ausführungsform der Klemmvorrichtung wird beim Einschrauben einer Schraube 3 in eine Plattenbohrung 15 durch die kegelstumpfförmige Ausgestaltung der Schraube 3 mit der dazu komplementär ausgestalteten, kegelförmigen Plattenbohrung 15 die Schraube 3 auf ihrem radial elastischen Segment der Länge L radial komprimiert.

Geeignete Bereiche der Abmessungen für die Schraube 3 und die dazu korrespondierenden Strukturen am Klemmring 11, bzw. an der Knochenplatte 14 für verschiedene Ausführungsformen sind nachfolgend aufgeführt:
- Höhe H der Schraube: 3 mm - 7 mm;
- Länge der Längsschlitze: 2,5 mm - 4,5 mm;
- Breite der Längsschlitze 0,3 mm -1,2 mm;
- Dicke des Schraubenmantels: konisch wachsend von 0,8 mm bis 4,0 mm
- Durchmesser der Durchgangsbohrung 1,0 mm - 3,0 mm;
- Spiel zwischen Durchgangsbohrung und Kirschnerdraht 0 bis 0,1 mm;
- Konizität 15° - 25 °;
- Steigung eines zweigängigen Gewindes 0,6 -1,1 mm, typischerweise 0,8 mm.

## Patentansprüche

1. Klemmvorrichtung zum Festklemmen eines longitudinalen Fixationselementes (2), insbesondere eines Kirschnerdrahtes, bestehend aus einer mindestens teilweise kegelstumpfförmig ausgebildeten Schraube (3) der Bauhöhe H mit einer Kegelachse (4) und einer kegelförmigen Mantelfläche (5) mit einem Gewinde (6), einer koaxial zur Kegelachse (4) verlaufenden Durchgangsbohrung (7) zur Aufnahme des longitudinalen Fixationselementes (2), einem hinteren Ende (8) mit Mitteln (9) zum Eindrehen der Schraube (3) und einem vorderen Ende (1), wobei das vordere Ende (1) der Schraube (3) mit mindestens einem Längsschlitz (10) der Länge L versehen ist, welcher radial bis in die Durchgangsbohrung (7) verläuft, so dass die Schraube (3) mindestens teilweise radial komprimierbar ist,
**dadurch gekennzeichnet, dass**
A) die Klemmvorrichtung eine Knochenplatte (14) mit mehreren Plattenbohrungen (15) umfasst, wobei
B) mindestens eine der Plattenbohrungen (15) kegelförmig ist und geeignet ist, die Schraube (3) aufzunehmen und mindestens im Bereich des vorderen Endes (1) radial zu komprimieren und wobei
C) die mindestens eine kegelförmige Plattenbohrung (15) ein konisches Innengewinde (16;20) aufweist, das komplementär zum Gewinde (6) der Mantelfläche (5) der Schraube (3) ausgestaltet ist.

2. Klemmvorrichtung nach Anspruch 1, **dadurch gekennzeichnet, dass** das vordere Ende (1) der Schraube (3) mit drei oder vier Längsschlitzen (10) versehen ist, welche vorzugsweise in gleichmässigen Abständen angeordnet sind.

3. Klemmvorrichtung nach Anspruch 1 oder 2, **dadurch gekennzeichnet, dass** die Länge L der Längsschlitze (10) kleiner ist als die Bauhöhe H der Schraube (3) und vorzugsweise L < 0,8 H gilt.

## Claims

1. A clamping device to firmly clamp a longitudinal fixing element (2), in particular a Kirschner wire, comprising a screw (3) with an at least partly truncated cone shape having an overall height *H* with a taper axis (4) and a taper-shaped jacket surface (5) with a thread (6), a through bore (7) extending coaxially with the taper axis (4) to accommodate the longitudinal fixing element (2), a rear end (8) with means (9) to screw in the screw (3) and a front end (1), wherein the front end (1) of the screw (3) is provided with at least one longitudinal slot (10) with a length *L*, the slot radially extending up to the through bore (7), so that the screw (3) can be compressed at least partly in the radial direction,
**characterized in that**
A) the clamping device comprises a bone plate (14) with a plurality of plate holes (15),
wherein
B) at least one of the plate holes (15) is tapered and suitable to accommodate the screw (3) and radially compress it at least in the region of the front end (1), and wherein
C) the at least one tapered plate hole (15) has a tapered internal thread (16;20) that is complementarily configured to the thread (6) of the jacket surface (5) of the screw (3).

2. A clamping device according to claim 1, **characterised in that** the front end (1) of the screw (3) is provided with three or four longitudinal slots (10), that are preferably equidistant from one another.

3. A clamping device according to claim 1 or 2, **characterised in that** the length L of the longitudinal slots (10) is smaller than the overall height *H* of the screw (3) and preferably *L* < 0.8 *H*.

## Revendications

1. Dispositif de serrage servant à immobiliser un élément de fixation longitudinal (2), en particulier une broche de Kirschner, se composant d'une vis (3), d'une hauteur prévue H, configurée au moins partiellement en forme de tronc de cône et comportant un axe de cône (4) et une surface latérale conique (5) comprenant un filetage (6), d'un trou traversant (7) s'étendant de façon coaxiale par rapport à l'axe de cône (4) et servant au logement de l'élément de fixation longitudinal (2), se composant d'une extrémité arrière (8) comportant des moyens (9) servant à visser la vis (3), et d'une extrémité avant (1) où l'extrémité avant (1) de la vis (3) est dotée d'au moins une fente longitudinale (10), d'une longueur L, qui s'étend radialement à l'intérieur du trou traversant (7), de sorte que la vis (3) peut être comprimée au moins partiellement dans le sens radial,
**caractérisé en ce que**
A) le dispositif de serrage comprend une plaque (14) pour ostéosynthèse, comportant plusieurs trous de plaque (15), où
B) au moins l'un des trous de plaque (15) est de forme conique et est approprié pour loger la vis (3) et pour la comprimer radialement au moins dans la zone de l'extrémité avant (1), et où
C) le trou de plaque (15) de forme conique, au moins au nombre de un, comporte un filetage intérieur conique (16 ; 20) qui est configuré en complément du filetage (6) de la surface latérale (5) de la vis (3).

2. Dispositif de serrage selon la revendication 1, **caractérisé en ce que** l'extrémité avant (1) de la vis (3) est dotée de trois ou quatre fentes longitudinales (10) qui sont disposées, de préférence, à des distances égales.

3. Dispositif de serrage selon la revendication 1 ou 2, **caractérisé en ce que** la longueur L des fentes longitudinales (10) est inférieure à la hauteur prévue H de la vis (3) et, de préférence, L < 0,8 H.
